(19) Europäisches Patentamt
European Patent Office
Office européen des brevets

(11) **EP 3 440 983 B1**

(12) **EUROPÄISCHE PATENTSCHRIFT**

(45) Veröffentlichungstag und Bekanntmachung des Hinweises auf die Patenterteilung:
**25.11.2020 Patentblatt 2020/48**

(51) Int Cl.:
*A47L 15/00* *(2006.01)*          *A61L 2/00* *(2006.01)*

(21) Anmeldenummer: **17185763.4**

(22) Anmeldetag: **10.08.2017**

(54) **VERFAHREN ZUR STEUERUNG DER DAUER EINER TROCKNUNGSPHASE EINES REINIGUNGSVERFAHRENS UND DAFÜR GEEIGNETES GERÄT**

METHOD OF CONTROLLING THE DURATION OF DRYING PHASE OF A CLEANING PROCESS AND DEVICE FOR SAME

PROCÉDÉ DE COMMANDE DE LA DURÉE D'UNE PHASE DE SÉCHAGE D'UN PROCÉDÉ DE NETTOYAGE ET APPAREIL DE NETTOYAGE APPROPRIÉ ASSOCIÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.02.2019 Patentblatt 2019/07**

(73) Patentinhaber: **MELAG Medizintechnik GmbH & Co. KG**
**10829 Berlin (DE)**

(72) Erfinder:
- **Halbich, Johannes**
**10405 Berlin (DE)**
- **Litzba, Guido**
**14513 Teltow (DE)**
- **Möller, Janis**
**22301 Hamburg (DE)**

(74) Vertreter: **Maikowski & Ninnemann**
**Patentanwälte Partnerschaft mbB**
**Postfach 15 09 20**
**10671 Berlin (DE)**

(56) Entgegenhaltungen:
WO-A1-2004/019750      CN-A- 105 105 694
JP-A- H01 181 832      JP-A- 2011 200 392

- **NADINA BAGHINA ET AL: "Predictive control of a domestic freezer for real-time demand response applications", INNOVATIVE SMART GRID TECHNOLOGIES (ISGT EUROPE), 2012 3RD IEEE PES INTERNATIONAL CONFERENCE AND EXHIBITION ON, IEEE, 14. Oktober 2012 (2012-10-14), Seiten 1-8, XP032332811, DOI: 10.1109/ISGTEUROPE.2012.6465809 ISBN: 978-1-4673-2595-0**

## Beschreibung

Die vorliegende Erfindung betrifft ein Verfahren zur Steuerung der Dauer einer Trocknungsphase eines Verfahrens zur Reinigung und optionalen Desinfektion von Reinigungsgut gemäß dem Oberbegriff des Anspruchs 1 sowie ein Gerät, das im Betrieb ein solches Verfahren umsetzt, gemäß dem Oberbegriff des Anspruchs 4.

Reinigungsgeräte oder Reinigungs- und Desinfektionsgeräte enthalten häufig ein integriertes Heizgebläse zur aktiven Trocknung der Beladung, da diese im Anschluss an den vorangegangenen Reinigungs- oder Desinfektionszyklus mit Wassertropfen benetzt ist. Für die aktive Trocknung wird Luft von einem Gebläse aus der Geräteumgebung angesaugt, mit Hilfe einer Heizung erhitzt und in das Innere einer Waschkammer des Geräts geleitet. Die auf dem Reinigungsgut (der sogenannten Beladung) befindlichen Tropfen verdunsten, und die Trocknungsluft reichert sich mit Feuchtigkeit an.

Am Ende der aktiven Trocknung ist die Wassermenge auf der Beladung gegenüber dem anfänglich vorliegenden Zustand reduziert. Allerdings verbleibt immer eine gewisse Restfeuchte auf der Beladung. Das Ausmaß der Restfeuchte ist von verschiedenen Faktoren abhängig, wie der Art und der Anzahl der zu trocknenden Beladungsgüter. Diese Faktoren erschweren es, ein für den Anwender reproduzierbares, zufriedenstellendes Trocknungsergebnis bei minimaler Trocknungszeit zu erzielen.

Aus dem Stand der Technik sind bereits einige Lösungen bekannt, die Trocknungszeit unter Berücksichtigung des tatsächlichen Feuchtigkeitsgrads der Beladung anzupassen.

Beispielsweise beschreibt das europäische Patent EP 1 689 280 B1 einen Geschirrspüler mit einem Feuchtigkeitssensor, der die Feuchtigkeit zumindest eines Teils der im Geschirrspüler vorhandenen Luft ermittelt. Der Trocknungsvorgang wird dann in Abhängigkeit von aktuell für die Feuchtigkeit ermittelten Messwerten des Feuchtigkeitssensors über eine elektronische Steuerung geregelt, damit die eingesetzten Wärmeerzeugungsmittel nur die erforderliche Wärmemenge produzieren.

Aus der DE 10 2008 043 176 A1 ist ein Trockner mit einem Zuluftkanal und einem Abluftkanal bekannt, die mittels eines Mechanismus über einen Umluftkanal zur Einstellung eines Anteils an Um- und Abluft im Trockner zusammengeschaltet werden können. Auf diese Weise lassen sich die Temperatur und die Feuchtigkeit in einem Innenraum des Trockners regeln. Dabei ist aus jener deutschen Patentanmeldung auch bekannt, einen Feuchtigkeitssensor zur Bestimmung der Feuchtigkeit der Luft des Raumes, in dem der Trockner aufgestellt ist, einzusetzen.

Aus der DE 10 2013 106 775 A1 ist ein Verfahren zum Trocknen von Spülgut in einem Geschirrspüler bekannt, bei der ein Differenzwert ermittelt wird, der einem ein Temperaturunterschied zwischen der Außenluft und der Luft in der Behandlungskammer des Geschirrspülers ermittelt wird. Auf der Grundlage dieses Temperaturunterschieds wird dann das Trocknungsverfahren gesteuert.

Aus der EP 0 953 315 A1 ist ein Verfahren zum Trocknen von Spülgut in einer Geschirrspülmaschine bekannt, bei dem die Luftfeuchtigkeit innerhalb des Spülbehälters überwacht wird, um eine Tür des Geschirrspülers automatisch zu öffnen, wenn ein Grenzwert der relativen Luftfeuchtigkeit erreicht wird. Dabei wird in jener europäischen Patentanmeldung auch angedacht, die Luftfeuchtigkeit des Aufstellortes der Geschirrspülmaschine zu erfassen, um einen Programmschritt des Trocknungsgangs zu beeinflussen. Es wird allerdings nicht erläutert, wie eine derartige Beeinflussung konkret erfolgen könnte.

Die WO 2004/019750 A1 beschreibt ein Verfahren zur Trocknung erwärmter Güter in einer Haushaltsmaschine. Zu Beginn des Trocknungsverfahrens wird dabei ein einziger Wert der Feuchtigkeit der Zuluft bestimmt. Dazu wird derselbe Feuchtigkeitssensor verwendet, der im Laufe des Trocknungsverfahrens auch die Feuchtigkeit der Abluft bestimmt. Eine zusätzliche Temperaturbestimmung ist gemäß der Lehre dieser internationalen Patentanmeldung nicht vorgesehen. Es ist auch nicht vorgesehen, die Feuchtigkeit anhand absoluter Feuchtigkeitswerte zu bestimmen.

Aus der CN 105105694 A1 ist ein Verfahren zur Trocknung von Reinigungsgut in einer Geschirrspülmaschine bekannt, bei dem ebenfalls zu Beginn des Verfahrens der Feuchtigkeitsgehalt der Umgebungsluft durch eine einzige Messung bestimmt wird. Diese Feuchtigkeit wird dann als zu erreichende Zielfeuchtigkeit vorgegeben. Mithin zielt jene chinesische Patentanmeldung darauf ab, die Feuchtigkeit im Inneren der Geschirrspülmaschine auf den am Anfang bestimmten Zielfeuchtigkeitswert einzustellen. Aus der JP 2011-200392 A ist ein Trocknungsverfahren bekannt, bei dem Zuluft zunächst in einem Entfeuchter vorgekühlt wird. Dadurch kondensiert Wasser, wodurch die Luftfeuchtigkeit der Zuluft sinkt. Anschließend wird die Zuluft wieder erwärmt und dann als getrocknete und erwärmte Luft zum Trocknen von Geschirr eingesetzt. Für die Überwachung der Lufttemperatur und des Erfolgs der Entfeuchtung werden gemäß der Lehre dieser japanischen Patentanmeldung zwei Temperatursensoren und zwei Feuchtigkeitssensoren eingesetzt. Diese Sensoren befinden sich alle im Lufteinlassbereich des Geräts. Die aus der Waschkammer austretende Abluft wird nicht in Bezug auf ihre Temperatur oder ihren Feuchtigkeitsgehalt überwacht.

Der vorliegenden Erfindung liegt die Aufgabe zugrunde, ein verbessertes Verfahren zur Steuerung der Dauer einer Trocknungsphase eines Verfahrens zur Reinigung und optionalen Desinfektion von Reinigungsgut anzugeben. Dabei sollen die Dauer der Trocknungsphase in Abhängigkeit der Art und der Menge des zu trocknenden Spülgutes auf das notwendige Minimum reduziert und ein stets reproduzierbares Trocknungsergebnis erzielt werden. Der vorliegenden Erfindung liegt ferner die Aufgabe zugrunde, eine Vorrichtung anzugeben, die ein derartiges Verfahren implementiert.

Diese Aufgabe wird durch ein Verfahren zur Steuerung der Dauer einer Trocknungsphase eines Verfahrens zur Reinigung und optionalen Desinfektion von Reinigungsgut mit den Merkmalen des Anspruchs 1 gelöst. Die Trocknungsphase kann die Trocknungsphase eines reinen Reinigungsverfahrens oder aber eines Reinigungs- und Desinfektionsverfahrens sein. Derartige Verfahren werden typischerweise in Reinigungsgeräten wie etwa Geschirrspülern oder in Reinigungs- und Desinfektionsgeräten durchgeführt. Sie dienen zur Reinigung und optionalen Desinfektion von Reinigungsgut wie etwa medizinischen oder zahnmedizinischen Instrumenten und Geräten.

Das erfindungsgemäß beanspruchte Verfahren zeichnet sich durch die nachfolgend erläuterten Schritte aus. In einem Verfahrensschritt wird regelmäßig eine Abluftfeuchtigkeit zu einem Zeitpunkt t bestimmt. Die Abluftfeuchtigkeit gibt dabei eine Feuchtigkeit von Abluft an, welche aus einer Reinigungskammer, in der das zuvor gereinigte und optional auch desinfizierte Reinigungsgut getrocknet wird, ausströmt.

In einem anderen Verfahrensschritt wird regelmäßig eine Zuluftfeuchtigkeit zum selben Zeitpunkt t bestimmt. Die Zuluftfeuchtigkeit gibt eine Feuchtigkeit von Zuluft an, die aus einer Umgebung der Reinigungskammer (insbesondere aus einer Umgebung des Reinigungsgeräts oder des Reinigungs- und Desinfektionsgeräts, in dem das Verfahren durchgeführt wird) angesaugt wird. Die Zuluft dient zum Trocknen des Reinigungsguts in der Reinigungskammer.

Darüber hinaus wird die Temperatur der Abluft, welche aus einer Reinigungskammer, in der das Reinigungsgut getrocknet wird, ausströmt, mittels eines ersten Temperatursensors bestimmt. Wenn die Temperatur zusätzlich zu der Feuchtigkeit bekannt ist, kann besonders einfach aus einem erhaltenen relativen Feuchtigkeitswert ein absoluter Feuchtigkeitswert berechnet werden, und umgekehrt. Das hat zur Folge, dass die Abluftfeuchtigkeit eine absolute Feuchtigkeit (auch als absolute Feuchte bezeichnet) der Abluft angibt.

In gleicher Weise wird die Temperatur von Zuluft, welche aus einer Umgebung der Reinigungskammer angesaugt und zum Trocknen des Reinigungsguts verwendet wird, mittels eines zweiten Temperatursensors bestimmt. Somit gibt die Zuluftfeuchtigkeit eine absolute Feuchtigkeit der Zuluft an.

Wenn eine Differenz zwischen der Abluftfeuchtigkeit und der Zuluftfeuchtigkeit einen vordefinierten Schwellenwert unterschreitet, wird die Trocknungsphase beendet. Denn dann ist das vorgebbare Trocknungskriterium erfüllt, sodass das Reinigungsgut den gewünschten Trocknungsgrad erreicht hat.

Bei dem vordefinierten Schwellenwert kann es sich um einen festen, invariablen Schwellenwert handeln. Alternativ kann der vordefinierte Schwellenwert auch aus einem vordefinierten Bereich oder einer vordefinierten Gruppe möglicher Schwellenwerte ausgewählt werden.

Zur Bildung der Differenz wird auf Messwerte zurückgegriffen, die sich auf den gleichen Zeitpunkt des Verfahrens beziehen. Eine solche Vorgehensweise erhöht die Genauigkeit.

In einer Variante ist der vordefinierte Schwellenwert aus einem Bereich von 0,1 bis 20 g/m$^3$ insbesondere von 0,2 bis 15 g/m$^3$, insbesondere von 0,3 bis 14 g/m$^3$, insbesondere von 0,4 bis 13 g/m$^3$, insbesondere von 0,5 bis 12 g/m$^3$, insbesondere von 0,6 bis 11 g/m$^3$, insbesondere von 0,7 bis 10 g/m$^3$, insbesondere von 0,8 bis 9 g/m$^3$, insbesondere von 0,9 bis 8 g/m$^3$, insbesondere von 1 bis 7 g/m$^3$, insbesondere von 2 bis 6 g/m$^3$, insbesondere von 3 bis 5 g/m$^3$ absoluter Feuchtigkeit ausgewählt. Dann gibt der Schwellenwert einen Wasserdampfgehalt von 0,1 bis 20 g pro Kubikmeter Luft bzw. einen entsprechenden Wasserdampfgehalt in einem der anderen vorgenannten Intervalle an. Ein aus dem Bereich von 0,1 bis 10 g/m$^3$ ausgewählter Schwellenwert eignet sich insbesondere für Reinigungskammern mit einem Nutzvolumen von bis zu 50 Litern. Ein aus dem Bereich von 0,1 bis 20 g/m$^3$ ausgewählter Schwellenwert eignet sich insbesondere für Reinigungskammern mit einem Nutzvolumen von mehr als 50 und bis zu 100 Litern. Ein besonders geeigneter Bereich zur Auswahl des Schwellenwertes ist der Bereich von 1 bis 5 g/m$^3$. Alternativ kann der Schwellenwert auch aus Werten relativer Feuchtigkeit ausgewählt werden, die den vorgenannten Werten der absoluten Feuchtigkeit entsprechen. Die relative Feuchtigkeit hängt dabei von der Temperatur der Abluft bzw. der Temperatur der Zuluft ab.

Wenn die Differenz zwischen der Abluftfeuchtigkeit und der Zuluftfeuchtigkeit kleiner als ein Wert ist, der innerhalb des vorgenannten Bereiches liegt, ist das Reinigungsgut hinreichend trocken, sodass eine weitere Trocknung nicht erforderlich ist. Diese würde auch wegen der geringen Differenz zwischen der Feuchtigkeit der Zuluft und der Feuchtigkeit der Abluft kaum für eine verbesserte Trocknung sorgen, gleichwohl aber eine Verlängerung der gesamten Trocknungsphase und eine Erhöhung der für die Trocknungsphase erforderlichen Energie zur Folge haben.

Grundsätzlich ist es möglich, die Differenz nicht anhand aktueller Messwerte für die Abluftfeuchtigkeit und die Zuluftfeuchtigkeit zum Zeitpunkt t zu berechnen, sondern auf der Grundlage von Messwerten für die Zuluftfeuchtigkeit und die Abluftfeuchtigkeit, die zu einem vorherigen Zeitpunkt gemessen wurden. Dann erfolgt zumindest die Berechnung eines Erwartungswertes der Abluftfeuchtigkeit, der dann zur Ermittlung der Differenz zwischen der Abluftfeuchtigkeit und der Zuluftfeuchtigkeit verwendet wird. Beispielsweise kann die Differenz auf der Grundlage eines solchen Erwartungswertes der Abluftfeuchtigkeit und eines Erwartungswertes der Zuluftfeuchtigkeit berechnet werden, wobei als Erwartungswert der Zuluftfeuchtigkeit beispielsweise eine zuvor gemessene Zuluftfeuchtigkeit in unveränderter Weise verwendet werden kann.

Der Erwartungswert der Abluftfeuchtigkeit kann gemäß der nachfolgenden Formel berechnet werden:

$$F(t) = F_U + F_0 * x^{-\frac{t}{T_{1/2}}}$$

Dabei bezeichnen

F(t)  den Erwartungswert der Abluftfeuchtigkeit zum Zeitpunkt t,
$F_U$  die Zuluftfeuchtigkeit,
$F_0$  die Abluftfeuchtigkeit abzüglich der Zuluftfeuchtigkeit zum Zeitpunkt t = 0,
x  einen Faktor zwischen 1,5 und 3,
t  die Zeit und
$T_{1/2}$  die Halbwertszeit, bei der der Erwartungswert der Abluftfeuchtigkeit der Summe aus Fu und Fo/2 entspricht.

Der Faktor x kann dabei die Eulersche Zahl e sein. Der Faktor x kann auch 2 betragen. Dann entspricht die vorstehend genannte Formel einem klassischen Zerfallsgesetz. Da kleinere Abweichungen von diesen exakten Zahlenwerten zu einer lediglich geringfügig geringeren Genauigkeit der Bestimmung des Erwartungswertes der Abluftfeuchtigkeit führen würden, liegen derartige Abweichungen für einen Fachmann im Bereich der Äquivalenz.

Wie bereits oben erwähnt, ist es grundsätzlich möglich, dass die Zuluftfeuchtigkeit $F_U$ zum Zeitpunkt t anhand der Zuluftfeuchtigkeit Fu zu einem vorherigen Zeitpunkt abgeschätzt wird. Beispielsweise kann die Zuluftfeuchtigkeit $F_U$ zum Zeitpunkt t mittels der Zuluftfeuchtigkeit $F_U$ zum Zeitpunkt t = 0 abgeschätzt bzw. mit dieser gleichgesetzt werden.

Die Halbwertszeit $T_{1/2}$ kann messtechnisch ermittelt werden, also mittels einer kontinuierlichen Messung der Feuchtigkeit in der Abluft bzw. mittels einer kontinuierlichen Differenzbildung der Feuchtigkeit in Zu- und Abluft bestimmt werden.

Nicht beanspruchte Alternativen beinhalten, die Halbwertszeit $T_{1/2}$ auf einen vordefinierten Wert einzustellen. Dieser Wert kann beispielsweise aus einer Liste möglicher Halbwertszeiten ausgewählt werden, die zuvor für eine bestimmte Art des Reinigungsguts (also eine bestimmte Art der Beladung) bei einer bestimmten anfänglichen Differenz zwischen der Abluftfeuchtigkeit und der Zuluftfeuchtigkeit, insbesondere zum Zeitpunkt t = 0, ermittelt wurden. Aber es ist beispielsweise auch möglich, dass ein Benutzer die Art der Beladung vorgibt und dann automatisch aus der anfänglichen Differenz zwischen der Abluftfeuchtigkeit und der Zuluftfeuchtigkeit eine voraussichtliche Halbwertszeit ermittelt wird, die als Halbwertszeit in der oben stehenden Formel eingesetzt wird. Wenn die Halbwertszeit auf diese Weise festgelegt wurde und die Zuluftfeuchtigkeit $F_U$ (beispielsweise in Form der anfänglichen Zuluftfeuchtigkeit zum Zeitpunkt t = 0) gemessen wurde sowie die anfängliche Abluftfeuchtigkeit zum Zeitpunkt t = 0 gemessen wurde, lässt sich die erwartete Abluftfeuchtigkeit zu jedem beliebigen Zeitpunkt der Trocknungsphase berechnen. Dann ist es auf besonders einfache Weise möglich, einen ausreichend genauen Erwartungswert der Abluftfeuchtigkeit für einen bestimmten Zeitpunkt t zu bestimmen.

Der Zeitpunkt t = 0 muss dabei nicht notwendigerweise der unmittelbare Beginn der Trocknungsphase sein. Vielmehr ist es denkbar, den Zeitpunkt t = 0 auf einen Zeitpunkt zu setzen, der nach dem tatsächlichen Beginn der Trocknungsphase liegt. Denn es hat sich gezeigt, dass zu Beginn der Trocknungsphase aufgrund eines sich noch nicht eingestellten Gleichgewichts sowie aufgrund von Sensorartefakten nicht in jedem Fall verlässliche Messwerte zur Bestimmung der initialen Abluftfeuchtigkeit erhalten werden können. Daher kann der Zeitpunkt t = 0 einer Zeit entsprechen, die in einem Bereich von 1 Minute bis 10 Minuten, insbesondere 1,5 Minuten bis 9 Minuten, insbesondere 2 Minuten bis 8 Minuten, insbesondere 2,5 Minuten bis 7 Minuten, insbesondere 3 Minuten bis 6 Minuten, insbesondere 3,5 Minuten bis 5 Minuten, insbesondere 4 Minuten bis 4,5 Minuten und ganz besonders rund 3 Minuten (beispielsweise 2,5 Minuten bis 3,5 Minuten) nach dem tatsächlichen Start der Trocknungsphase liegt.

Es ist möglich, eine Zeitdauer zu berechnen, die zum Erreichen eines Werts der Abluftfeuchtigkeit (voraussichtlich) erforderlich ist, welcher den Schwellenwert unterschreitet. Dies lässt sich ebenfalls anhand der oben wiedergegebenen Formel realisieren, die zu diesem Zweck nach t aufgelöst wird. Dann ist der Zeitpunkt bekannt, bei dem die Differenz zwischen der Abluftfeuchtigkeit und der Zuluftfeuchtigkeit einen vordefinierten Schwellenwert unterschreitet. Diese Zeitdauer kann beispielsweise angezeigt werden, um einem Benutzer Informationen darüber zu geben, wie lange die Trocknungsphase dauern wird. Grundsätzlich ist es möglich, die Trocknungsphase zu beenden, wenn die berechnete Zeitdauer verstrichen ist. Es ist also möglich, was ausserhalb der beanspruchten Erfindung fällt - bereits zu Beginn der Trocknungsphase, sofern die Halbwertszeit $T_{1/2}$ bekannt ist oder hinreichend genau abgeschätzt werden kann, auf der Grundlage eines Messwert der Abluftfeuchtigkeit und eines Messwert der Zuluftfeuchtigkeit zu bestimmen, wie lang die Trocknungsphase (voraussichtlich) dauern wird. Die Trocknungsphase wird dann beendet, wenn die Differenz zwischen der berechneten, erwarteten Abluftfeuchtigkeit zum Zeitpunkt t und der berechneten oder abgeschätzten Zuluftfeuchtigkeit zum Zeitpunkt t einen vordefinierten Schwellenwert unterschreitet, die berechnete Zeitdauer also verstrichen ist.

Messergebnisse von Feuchtigkeitssensoren weisen häufig eine Abhängigkeit von anderen Umgebungseinflüssen, wie beispielsweise der Temperatur, auf. Man spricht in diesem Zusammenhang auch von einem Temperaturgang der Feuchtigkeitssensoren. In einer Variante der beanspruchten Erfindung wird ein Korrekturfaktor für einen Messwert der Abluftfeuchtigkeit und/oder der Zuluftfeuchtigkeit bestimmt. Dieser Korrekturfaktor wird dann auf den entsprechenden Messwert angewandt. Es resultiert ein korrigierter Messwert der Abluftfeuchtigkeit und/oder der Zuluftfeuchtigkeit, der genauer als der ursprüngliche Messwert ist. Der Korrekturfaktor kann dabei beispielsweise den Temperaturgang des zur Messung der Abluftfeuchtigkeit und/oder der Zuluftfeuchtigkeit eingesetzten Sensors kompensieren. Es können auch mehrere Korrekturfaktoren für unterschiedliche Umwelteinflüsse verwendet werden, um insgesamt genauere Messergebnisse für die Abluftfeuchtigkeit und/oder die Zuluftfeuchtigkeit zu erhalten. Dies ist insbesondere dann von Vorteil, wenn die Feuchtigkeitsdifferenz zwischen der Abluftfeuchtigkeit und der Zuluftfeuchtigkeit gering ist. Denn je geringer diese Differenz ist, desto geringer ist die Genauigkeit des zur Messung der Abluftfeuchtigkeit oder der Zuluftfeuchtigkeit verwendeten Sensors. Gerade bei einer geringen Restfeuchte (Abluftfeuchtigkeit minus Zuluftfeuchtigkeit) stoßen Feuchtigkeitssensoren an ihre physikalischen Grenzen, sodass entsprechende Messwerte nur noch bedingt zur Durchführung des vorliegend beanspruchten Verfahrens geeignet sind.

Die vorliegende Erfindung betrifft auch eine Vorrichtung zur Reinigung und optionalen Desinfektion von Reinigungsgut mit den Merkmalen des Anspruchs 4. Bei einer solchen Vorrichtung kann es sich beispielsweise um ein Reinigungsgerät wie einen Geschirrspüler oder um ein Reinigungs- und Desinfektionsgerät handeln, das beispielsweise zur Reinigung und Desinfektion von ärztlichen oder zahnärztlichen Instrumenten und Geräten verwendet wird. Die Vorrichtung weist eine Reinigungskammer zur Aufnahme von Reinigungsgut auf. Darüber hinaus sind ein erster Feuchtigkeitssensor, ein zweiter Feuchtigkeitssensor und ein Steuergerät vorgesehen.

Die Vorrichtung zeichnet sich erfindungsgemäß dadurch aus, dass der erste Feuchtigkeitssensor zur Messung einer Abluftfeuchtigkeit dient. Die Abluftfeuchtigkeit gibt dabei die Feuchtigkeit von aus der Reinigungskammer austretender Abluft an. Der zweite Feuchtigkeitssensor dient zur Messung einer Zuluftfeuchtigkeit. Die Zuluftfeuchtigkeit gibt eine Feuchtigkeit von Zuluft an, die aus einer Umgebung der Reinigungskammer angesaugt wird. Ferner weist die Vorrichtung einen ersten Temperatursensor und einen zweiten Temperatursensor auf. Der erste Temperatursensor dient zur Messung einer Temperatur von aus der Reinigungskammer austretender Abluft. Der zweite Temperatursensor dient zur Messung einer Temperatur von aus einer Umgebung der Reinigungskammer angesaugter Zuluft. Durch den Einsatz derartiger Temperatursensoren lassen sich also neben der Feuchtigkeit der Abluft und der Zuluft auch die Temperatur der Abluft und der Zuluft bestimmen.

Das Steuergerät ist schließlich spezifisch dafür hergerichtet, im Betrieb der Vorrichtung das vorstehend beschriebene Verfahren durchzuführen. Mithin dient das Steuergerät insbesondere dafür, eine Differenz zwischen der Abluftfeuchtigkeit oder einem daraus berechneten Abluftfeuchtigkeitswert einerseits und der Zuluftfeuchtigkeit oder einem daraus berechneten Zuluftfeuchtigkeitswert andererseits zu berechnen und eine Trocknungsphase eines von der Vorrichtung durchgeführten Verfahrens zur Reinigung und optionalen Desinfektion von Reinigungsgut, das in der Reinigungskammer aufgenommen ist, zu beenden, wenn die Differenz einen vordefinierten Schwellenwert unterschreitet.

Bei dem ersten Feuchtigkeitssensor und/oder dem zweiten Feuchtigkeitssensor handelt es sich in einer Variante um ein kapazitives Hygrometer, ein Absorptionshygrometer, ein Psychrometer, ein Taupunktspiegelhygrometer, ein optisches Hygrometer, ein resistives Hygrometer oder ein coulometrisches Hygrometer. Dabei können der erste Feuchtigkeitssensor und der zweite Feuchtigkeitssensor unabhängig voneinander durch einen der vorgenannten Feuchtigkeitssensortypen realisiert werden. In einer Variante sind der erste Feuchtigkeitssensor und der zweite Feuchtigkeitssensor baugleich.

In einer Variante weist die Vorrichtung einen ersten Drucksensor auf, der zur Messung eines Luftdrucks innerhalb der Reinigungskammer oder von aus der Reinigungskammer austretender Abluft dient, und einen zweiten Drucksensor, der zur Messung eines Luftdrucks von aus einer Umgebung der Reinigungskammer angesaugter Zuluft dient. Dann lassen sich weitere Messdaten generieren, die beispielsweise zur Erhöhung der Genauigkeit der Messergebnisse des ersten Feuchtigkeitssensors und/oder des zweiten Feuchtigkeitssensors verwendet werden können.

Sämtliche Varianten und Ausgestaltungen des vorstehend beschriebenen Verfahrens sind in analoger Weise auf die vorstehend beschriebene Vorrichtung anwendbar, und umgekehrt. Dabei ist eine beliebige Kombination der erläuterten Varianten des Verfahrens und der Vorrichtung möglich.

Weitere Einzelheiten von Aspekten der der vorliegenden Erfindung werden anhand eines Ausführungsbeispiels und entsprechender Figuren näher erläutert. Es zeigen:

Figur 1 ein erstes Diagramm der absoluten Luftfeuchtigkeit in Abhängigkeit von der Zeit während einer Trocknungsphase eines Reinigungs- und Desinfektionsprozesses;

Figur 2 ein zweites Diagramm der absoluten Luftfeuchtigkeit in Abhängigkeit von der Zeit während einer Trocknungsphase eines Reinigungs- und Desinfektionsprozesses und

Figur 3 ein drittes Diagramm der absoluten Luftfeuchtigkeit in Abhängigkeit von der Zeit während einer Trocknungsphase eines Reinigungs- und Desinfektionsprozesses.

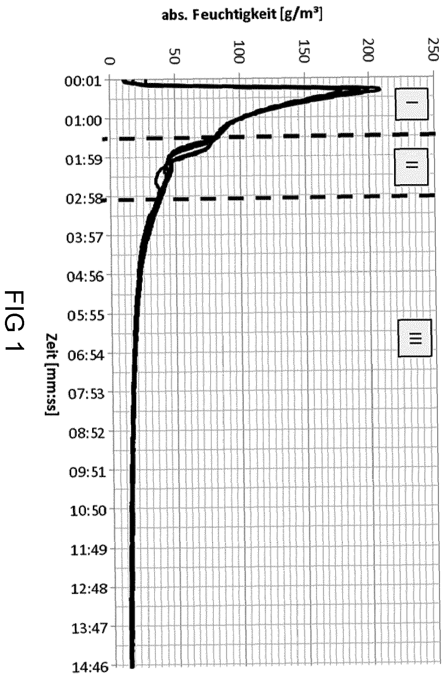

Die Figur 1 zeigt einen beispielhaften Verlauf der absoluten Feuchtigkeit der aus einer Reinigungskammer austretenden Abluft während der Trocknungsphase eines Reinigungs- und Desinfektionsprozesses von zahnmedizinischen Instrumenten und Geräten. Dabei sind die Feuchtigkeitskurven von fünf Wiederholversuchen übereinander gelagert.

Der Verlauf der absoluten Feuchtigkeit mit der Zeit lässt sich in drei Phasen gliedern, die in der Figur 1 mit I, II und III bezeichnet sind. In der Phase 1 beträgt die relative Luftfeuchtigkeit der Abluft in etwa 100 %. Durch eine Erhöhung der Temperatur der Abluft zu Beginn des Trocknungsprozesses steigt die absolute Feuchtigkeit an. Anschließend fällt sie jedoch ab, da nun eine tatsächliche Trocknung der Beladung einsetzt. Die Phase I dauert im Beispiel der Figur 1 rund 1,5 Minuten und geht dann in die Phase II über.

Der Feuchtigkeitsverlauf in der Phase II weist zwischen den einzelnen Wiederholversuchen sehr große Varianzen auf. Dies ist auf Messartefakte bei der Bestimmung der absoluten Feuchtigkeit zurückzuführen. Im Bereich von rund 3 Minuten nach Beginn der Trocknungsphase geht die Phase II in die Phase III über, in der die Feuchtigkeitswerte sämtlicher Experimente mit sehr geringer Varianz übereinanderliegen. Die absolute Feuchtigkeit der Abluft nähert sich dabei der absoluten Feuchtigkeit der Zuluft an. Ab einem Zeitpunkt von rund 10 Minuten nach Beginn der Trocknungsphase lässt sich praktisch keine weitere Reduzierung der absoluten Feuchtigkeit der Abluft mehr erreichen. Zu diesem Zeitpunkt beträgt Differenz zwischen der absoluten Feuchtigkeit der Abluft und der absoluten Feuchtigkeit der der Reinigungskammer zugeführte Zuluft nur noch 1 bis 5 g/m$^3$.

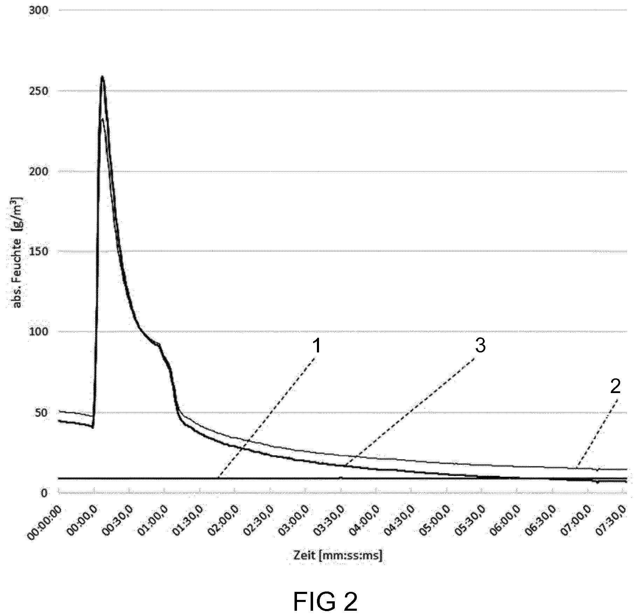

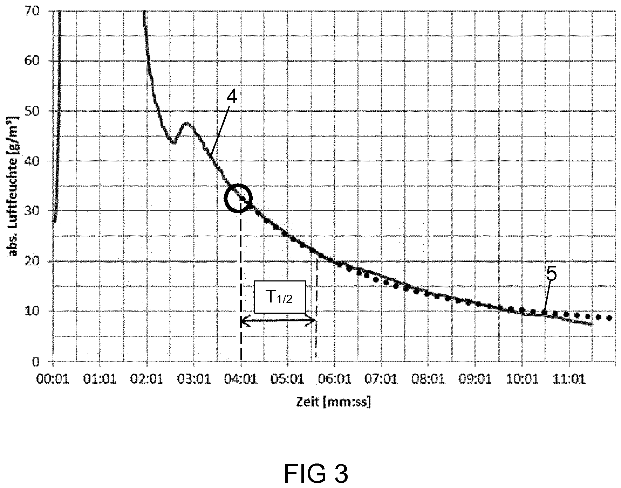

Den Verlauf der absoluten Feuchtigkeit kann man auch anhand des in der Figur 2 dargestellten Diagramms ersehen. So zeigt die Figur 2 den Verlauf der absoluten Feuchtigkeit 1 in der Zuluft, den Verlauf der absoluten Feuchtigkeit 2 in der Abluft und die Differenz 3 zwischen der absoluten Feuchtigkeit 2 in der Abluft und der absoluten Feuchtigkeit 1 in der Zuluft. Wie aus der Figur 2 gut ersichtlich ist, ändert sich die absolute Feuchtigkeit 1 in der Zuluft während der Trocknungsphase kaum. Demgegenüber nimmt die absolute Feuchtigkeit 2 in der Abluft während der Trocknungsphase ab und erreicht bei etwa 5,5 Minuten den Wert der absoluten Feuchtigkeit in der Zuluft. Nachfolgend ist keine weitere relevante Trocknung der in der Reinigungskammer des Reinigungs- und Desinfektionsgeräts enthaltenen Beladung möglich. Die Figur 3 zeigt den Verlauf der absoluten Feuchtigkeit 4 der aus der Reinigungskammer eines Reinigungs- und Desinfektionsgeräts austretenden Abluft während der Trocknungsphase eines Reinigungs- und Desinfektionsverfahrens. Darüber hinaus ist gepunktet eine gemäß der Formel $$F(t) = F_U + F_0 * 2^{-\frac{t}{T_{1/2}}}$$ berechnete Kurve 5 dargestellt, deren Startwert t = 0 auf einen Zeitpunkt von 4 Minuten nach Beginn der Trocknungsphase gesetzt wurde. Zu diesem Zeitpunkt befindet sich die Trocknung in der in der Figur 1 dargestellten Phase III, weist also eine einen steten Verlauf mit geringer Varianz auf. Zu diesem Zeitpunkt t = 0 beträgt die Feuchtigkeit der Abluft $F_0$ + Fu. Nach einer Halbwertszeit $T_{1/2}$ von rund 2 Minuten ist die Abluftfeuchtigkeit auf $F_0$/2 + Fu gesunken. Der Verlauf der berechneten Feuchtigkeit 5 stimmt sehr gut mit dem Verlauf der tatsächlich gemessenen Feuchtigkeit 4 überein, sodass nach der Ermittlung eines initialen Wertes für $F_0$ und $F_U$ keine weitere Messung der absoluten Feuchtigkeit der Abluft oder der Zuluft erforderlich ist. Vielmehr kann dann, wenn die Halbwertszeit $T_{1/2}$ bekannt oder vorgegeben ist, ein erwarteter Verlauf der absoluten Luftfeuchtigkeit der Abluft berechnet werden, der sehr gut mit dem tatsächlich berechneten gemessenen Verlauf übereinstimmt. Aus einer Differenz des derart berechneten Werts der absoluten Abluftfeuchtigkeit und der eingangs gemessenen Zuluftfeuchtigkeit lässt sich dann bestimmen, ob die Trocknungsphase beendet werden kann. Dies ist dann möglich, wenn die vorgenannte Differenz unter einen vordefinierten Grenzwert gesunken ist.

## Patentansprüche

1. Verfahren zur Steuerung der Dauer einer Trocknungsphase eines Verfahrens zur Reinigung und optionalen Desinfektion von Reinigungsgut, mit den folgenden Schritten:

   a) regelmäßiges Messen eines Feuchtigkeitswerts einer Abluftfeuchtigkeit (2, 4) zu einem Zeitpunkt t unter Verwendung eines ersten Feuchtigkeitssensors,
   b) Bestimmen der Temperatur von Abluft, welche aus einer Reinigungskammer, in der das Reinigungsgut getrocknet wird, ausströmt, mittels eines ersten Temperatursensors, so dass die Abluftfeuchtigkeit (2, 4) eine absolute Feuchtigkeit der Abluft angibt,
   c) regelmäßiges Messen eines Feuchtigkeitswerts einer Zuluftfeuchtigkeit (1) zum selben Zeitpunkt t unter Verwendung eines zweiten Feuchtigkeitssensors, wobei zusätzlich die Temperatur von Zuluft, welche aus einer Umgebung der Reinigungskammer angesaugt und zum Trocknen des Reinigungsguts verwendet wird, mittels eines zweiten Temperatursensors bestimmt wird, so dass die Zuluftfeuchtigkeit (1) eine absolute Feuchtigkeit der Zuluft angibt, und
   d) Beenden der Trocknungsphase, wenn eine Differenz (3) zwischen der Abluftfeuchtigkeit (2, 4) und der Zuluftfeuchtigkeit (1) einen vordefinierten Schwellenwert unterschreitet.

2. Verfahren nach Anspruch 1, **dadurch gekennzeichnet, dass** der vordefinierte Schwellenwert aus einem Bereich von 0,1 bis 20 g/m$^3$ absoluter Feuchtigkeit ausgewählt ist.

3. Verfahren nach einem der vorherigen Ansprüche, **dadurch gekennzeichnet, dass** ein Korrekturfaktor für einen Messwert der Abluftfeuchtigkeit (2, 4) und/oder der Zuluftfeuchtigkeit (1) bestimmt und auf den entsprechenden Messwert angewandt wird, um einen korrigierten Messwert der Abluftfeuchtigkeit (2, 4) und/oder der Zuluftfeuchtigkeit (1) zu erhalten.

4. Vorrichtung zur Reinigung und optionalen Desinfektion von Reinigungsgut, mit einer Reinigungskammer zur Aufnahme von Reinigungsgut, einem ersten Feuchtigkeitssensor, einem zweiten Feuchtigkeitssensor und einem Steuergerät,
   **dadurch gekennzeichnet,**
   **dass** die Vorrichtung einen ersten Temperatursensor, der zur Messung einer Temperatur von aus der Reinigungskammer austretender Abluft dient, und einen zweiten Temperatursensor, der zur Messung einer Temperatur von aus einer Umgebung der Reinigungskammer angesaugter Zuluft dient, aufweist, dass der erste Feuchtigkeitssensor zur Messung eines Feuchtigkeitswerts einer Abluftfeuchtigkeit (2, 4) dient, die eine Feuchtigkeit von aus der Reinigungskammer austretender Abluft angibt, dass der zweite Feuchtigkeitssensor zur Messung eines Feuchtigkeitswerts einer Zuluftfeuchtigkeit (1) dient, die eine Feuchtigkeit von aus einer Umgebung der Reinigungskammer angesaugter Zuluft angibt, und dass das Steuergerät dafür vorgesehen und eingerichtet ist, das Verfahren gemäß einem der Ansprüche 1 bis 3 auszuführen.

5. Vorrichtung nach Anspruch 4, **dadurch gekennzeichnet, dass** der erste Feuchtigkeitssensor und/oder der zweite Feuchtigkeitssensor unabhängig voneinander ausgewählt sind aus der Gruppe bestehend aus kapazitiven Hygrometern, Absorptionshygrometern, Psychrometern, Taupunktspiegelhygrometern, optischen Hygrometern, resistiven Hygrometern und coulometrischen Hygrometern.

**Claims**

1. Method of controlling the duration of a drying phase of a method for cleaning and optionally disinfecting items to be cleaned, comprising the following steps:

   a) regularly measuring a humidity value of an exhaust air humidity (2, 4) at a time t by using a first humidity sensor,
   b) determining the temperature of exhaust air flowing out of a cleaning chamber, in which the items to be cleaned are dried, by means of a first temperature sensor so that the exhaust air humidity (2, 4) indicates an absolute humidity of the exhaust air,
   c) regularly measuring a humidity value of a supply air humidity (1) at the same time t by using a second humidity sensor, wherein in addition the temperature of supply air, which is sucked in from an environment of the cleaning chamber and is used for drying the items to be cleaned, is determined by means of a second temperature sensor so that the supply air humidity (1) indicates an absolute humidity of the supply air, and
   d) terminating the drying phase when a difference (3) between the exhaust air humidity (2, 4) and the supply air humidity (1) falls below a predefined threshold value.

2. The method according to claim 1, **characterized in that** the predefined threshold value is selected from a range of 0.1 to 20 g/m$^3$ of absolute humidity.

3. The method according to any of the preceding claims, **characterized in that** a correction factor for a measured value of the exhaust air humidity (2, 4) and/or the supply air humidity (1) is determined and applied to the corresponding measured value in order to obtain a corrected measured value of the exhaust air humidity (2, 4) and/or the supply air humidity (1).

4. An apparatus for cleaning and optionally disinfecting items to be cleaned, comprising a cleaning chamber for accommodating items to be cleaned, a first humidity sensor, a second humidity sensor and a control unit,
   **characterized in**
   **that** the apparatus includes a first temperature sensor, which serves for measuring a temperature of exhaust air exiting from the cleaning chamber, and a second temperature sensor, which serves for measuring a temperature of supply air sucked in from an environment of the cleaning chamber, that the first humidity sensor serves for measuring a humidity value of an exhaust air humidity (2, 4) which indicates a humidity of exhaust air exiting from the cleaning chamber, that the second humidity sensor serves for measuring a humidity value of a supply air humidity (1) which indicates a humidity of supply air sucked in from an environment of the cleaning chamber, and that the control unit is provided and equipped to carry out the method according to any of claims 1 to 3.

**5.** The apparatus according to claim 4, **characterized in that** the first humidity sensor and/or the second humidity sensor are independently selected from the group including capacitive hygrometers, absorption hygrometers, psychrometers, mirror-type dew point hygrometers, optical hygrometers, resistive hygrometers and coulometric hygrometers.

## Revendications

**1.** Procédé pour commander la durée d'une phase de séchage d'un procédé de nettoyage et de désinfection en option de produits de nettoyage, avec les étapes suivantes :

a) de mesure régulière d'une valeur d'humidité d'une humidité d'air sortant (2, 4) à un moment t en utilisant un premier capteur d'humidité,
b) de définition de la température d'un air sortant, lequel s'écoule hors d'une chambre de nettoyage, dans laquelle le produit de nettoyage est séché, au moyen d'un premier capteur de température de sorte que l'humidité d'air sortant (2, 4) indique une humidité absolue de l'air sortant,
c) de mesure régulière d'une valeur d'humidité d'une humidité d'air entrant (1) au même moment t en utilisant un deuxième capteur d'humidité, dans lequel en supplément la température de l'air entrant, lequel est aspiré depuis un extérieur de la chambre de nettoyage et est utilisé pour sécher le produit de nettoyage, est définie au moyen d'un deuxième capteur de température de sorte que l'humidité d'air entrant (1) indique une humidité absolue de l'air entrant, et
d) de fin de la phase de séchage quand une différence (3) entre l'humidité d'air sortant (2, 4) et l'humidité d'air entrant (1) reste inférieure à une valeur de seuil prédéfinie.

**2.** Procédé selon la revendication 1, **caractérisé en ce que** la valeur de seuil prédéfinie est choisie parmi une plage de 0,1 à 20 $g/m^3$ d'humidité absolue.

**3.** Procédé selon l'une quelconque des revendications précédentes, **caractérisé en ce qu'**un facteur de correction est défini pour une valeur de mesure de l'humidité d'air sortant (2, 4) et/ou de l'humidité d'air entrant (1) et est appliqué sur la valeur de mesure correspondante pour obtenir une valeur de mesure corrigée de l'humidité d'air sortant (2, 4) et/ou de l'humidité d'air entrant (1).

**4.** Dispositif de nettoyage et de désinfection en option de produit de nettoyage, avec une chambre de nettoyage pour recevoir du produit de nettoyage, un premier capteur d'humidité, un deuxième capteur d'humidité et un appareil de commande, **caractérisé en ce que** le dispositif présente un premier capteur de température, qui sert à la mesure d'une température d'air sortant sortant de la chambre de nettoyage, et un deuxième capteur de température, qui sert à la mesure d'une température d'air entrant aspiré depuis un extérieur de la chambre de nettoyage, que le premier capteur d'humidité sert à la mesure d'une valeur d'humidité d'une humidité d'air sortant (2, 4), qui indique une humidité d'air sortant sortant de la chambre de nettoyage, que le deuxième capteur d'humidité sert à la mesure d'une valeur d'humidité d'une humidité d'air entrant (1), qui indique une humidité d'air entrant aspiré depuis un extérieur de la chambre de nettoyage, et que l'appareil de commande est prévu et mis au point pour exécuter le procédé selon l'une quelconque des revendications 1 à 3.

**5.** Dispositif selon la revendication 4, **caractérisé en ce que** le premier capteur d'humidité et/ou le deuxième capteur d'humidité sont choisis indépendamment l'un de l'autre parmi le groupe constitué d'hygromètres capacitifs, d'hygromètres d'absorption, de psychromètres, d'hygromètres de miroir à point de rosée, d'hygromètres optiques, d'hygromètres résistifs et d'hygromètres coulométriques.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1689280 B1
- DE 102008043176 A1
- DE 102013106775 A1
- EP 0953315 A1
- WO 2004019750 A1
- CN 105105694 A1
- JP 2011200392 A